# EUROPEAN PATENT SPECIFICATION

(11) **EP 0 595 499 B1**
(45) Date of publication and mention of the grant of the patent: **09.09.1998**
(21) Application number: 93308084.8
(22) Date of filing: 11.10.1993
(51) Int. Cl.: A61M 1/00

(54) **Suction catheter assemblies**
Saugkatheteraufbauten
Assemblages de cathéters de succion

(30) Priority: 28.10.1992 US 967844; 25.06.1993 US 82016
(43) Date of publication of application: 04.05.1994
(73) Proprietor: SIMS PORTEX, INC., Keene, New Hampshire 03431 (US)
(72) Inventor: Devlin, Thomas, Cambridge MA 02139 (US); Ulrich, Karl, Belmont, MA 02178 (US)
(74) Representative: Flint, Jonathan McNeill

(56) References cited:
- GB-A- 2 207 736
- US-A- 5 073 164

## Description

This invention relates to suction catheter assemblies of the kind for use in removing undesirable fluid from a patient, the catheter assembly including an aspirating catheter having a distal end to be inserted in a patient, a vacuum connecting member located in the vicinity of the proximal end of the aspirating catheter, a patient connecting member surrounding the aspirating catheter in the vicinity of the distal end of the aspirating catheter and having a sliding seal with the outside of the aspirating catheter, a protective sleeve surrounding at least the majority of the length of the catheter and extending between the vacuum connecting member and the patient connecting member, the protective sleeve permitting the distal end of the catheter to be extended from the protective sleeve into the patient and to be withdrawn from the patient, and the patient connecting member including a one-way valve permitting gas flow through it from inside the sleeve and into the patient connecting member but preventing any substantial gas flow through the one-way valve into the sleeve such that any gas trapped in the sleeve can escape into the patient connecting member through the one-way valve.

The coupling has one port connected to a tracheal tube and two further side ports by which ventilation of the patient can take place. In use, the machine end of the catheter is connected to a suction source via a valve. Secretions that build up on the inside of the tracheal tube, the trachea and bronchi can be periodically removed by opening the valve and advancing the catheter through the coupling and down the tracheal tube. The coupling enables ventilation of the patient to continue while suctioning takes place.

One problem with this kind of assembly is that air may enter the sleeve causing it to inflate which can make subsequent use of the assembly more difficult. A small amount of air will be present in the sleeve as a result of assembly and additional air can be pulled into the sleeve during the negative pressure cycle of sterilization. Air from the ventilation system, during use, can also be forced back into the sleeve. Although a sliding seal can be provided in the coupling with the outside of the catheter, this does not provide a total air seal. Attempts to improve the seal by making it a tighter fit tend to cause an indentation in the catheter, especially when it is stored for prolonged periods or subjected to elevated temperature, such as during sterilization. One way of preventing the accumulation of air in the sleeve is to provide a small vent that allows air to escape to atmosphere. This, however, is not desirable because it can allow the escape of contaminated material from the assembly onto the user.

Examples of catheter assemblies having an aspirating catheter which is contained within a sleeve and which can be pushed through a sliding seal on a coupling are described in several patents, such as US 3,991,752 to Radford; US 4,569,344 to Palmer; US 4,638,539 to Palmer; US 4,696,296 to Palmer; US 4,825,859 to Lambert; US 4,834,726 to Lambert; US 4,836,199 to Palmer; US 4,838,255 to Lambert; US 4,872,579 to Palmer; US 4,938,741 to Lambert; US 4,967,743 to Lambert; US 4,981,466 to Lambert; US 5,025,806 to Palmer; US 5,029,580 to Radford; US 5,060,646 to Page; US 5,065,754 to Jensen; US 5,073,164 to Hollister. GB 2207736 to Hollister describes a catheter assembly in which the sliding seal can act as a valve to allow gas to escape between the seal and the outside of the catheter. Suction catheter assemblies are also available from Smiths Industries Medical Systems Inc under the trade mark STERICATH and from Ballard Medical Products Inc under the trade mark TRACHCARE.

It is an object of the present invention to provide a suction catheter assembly in which there is a reduced risk of inflation of the sleeve and in which the user is protected from contact with contaminated material.

According to the present invention there is provided a suction catheter assembly of the above-specified kind, characterised in that the one-way valve has a one-way gas passage from inside the sleeve other than via the sliding seal and the outside of the catheter.

The one-way valve may take various different forms. In one form, the one-way valve is a duck-bill valve. The patient connecting member may include a main passage through which the aspirating catheter is advanced and a by-pass channel, the one-way valve being located in the by-pass channel. The by-pass channel may be substantially parallel with the main passage and may communicate with the main passage at one end at a location facing the sleeve and the rear of the sliding seal and at the other end at a location facing the front of the sliding seal. Alternatively, the sliding seal and one-way valve may be provided by a resilient tubular member having a flange at its proximal end and an internal wiper seal with the catheter at its distal end, the assembly including a shoulder that faces distally of the assembly, the proximal surface of the flange lying against the shoulder and the distal surface of the flange being exposed to gas pressure in the patient connecting member distally of the tubular member such that the flange is forced into closer contact with the shoulder by gas pressure on the distal side of the tubular member and is lifted away from the shoulder by gas pressure on the proximal side of the tubular member.

Alternatively, the one-way valve may include a disc with a central aperture through which the catheter extends, the disc having at least one peripheral aperture therethrough providing the one-way gas passage, and a displaceable member overlying a distal face of the disc in the region of said peripheral aperture such that gas pressure on the proximal side of the disc is effective to displace the displaceable member away from the peripheral aperture so as to allow gas flow through the peripheral aperture.

In another form of assembly, the one-way valve may include a porous disc providing the one-way gas passage, the disc having an aperture through which the catheter extends, and a flexible diaphragm overlying a distal face of the disc such that gas pressure on the proximal side of the disc is effective to displace apart of the diaphragm away from the disc so as to allow gas flow through the disc, and such that gas pressure on the distal side of the disc is effective to urge the diaphragm against the disc and prevent gas flow therethrough.

The patient connecting member preferably includes a patient end coupling aligned with the aspirating catheter, the patient end coupling being adapted to be coupled with a tracheal tube, the patient end coupling having two side ports communicating with the interior of the patient end coupling on the distal side of the one-way valve, and the two side ports being adapted for connection to ventilation apparatus such that the patient can be aspirated without interruption of ventilation. The vacuum connecting member may include a manually-operable valve, the manually-operable valve normally blocking fluid flow along the aspirating catheter but can be manually opened to permit fluid flow along the catheter.

Several suction catheter assemblies according to the present invention, will now be described, by way of example, with reference to the accompanying drawings, in which:
- Figure 1: is a perspective view of the assembly;
- Figure 2: is a sectional side elevation of the patient connecting member;
- Figure 3: is a transverse section along the line III-III of Figure 2;
- Figures 4 and 5: are sectional views of an alternative patient connecting member;
- Figures 6 to 8: are enlarged sectional views of alternative valves;
- Figure 9: is a transverse section along the line IX - IX of Figure 8;
- Figure 10: is an exploded, perspective view of still another embodiment of the patient connecting member; and
- Figure 11: is a transverse section taken along the line XI-XI of Figure 10.

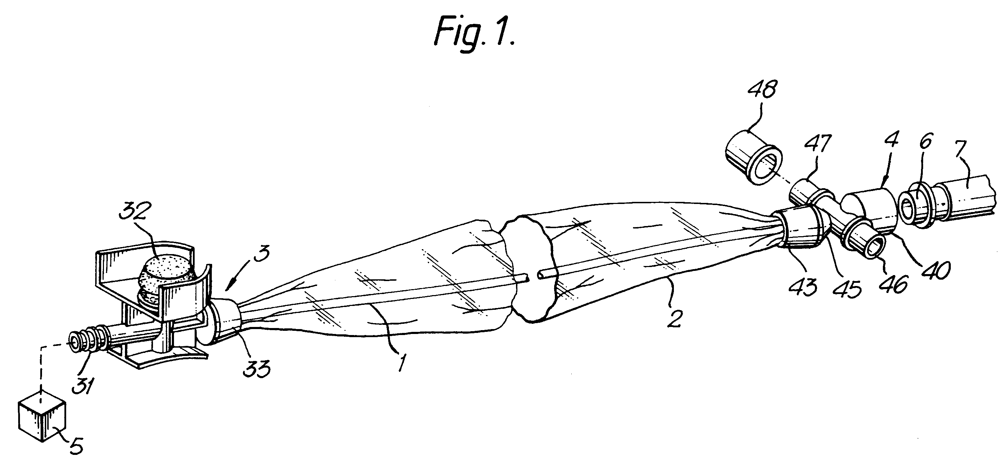

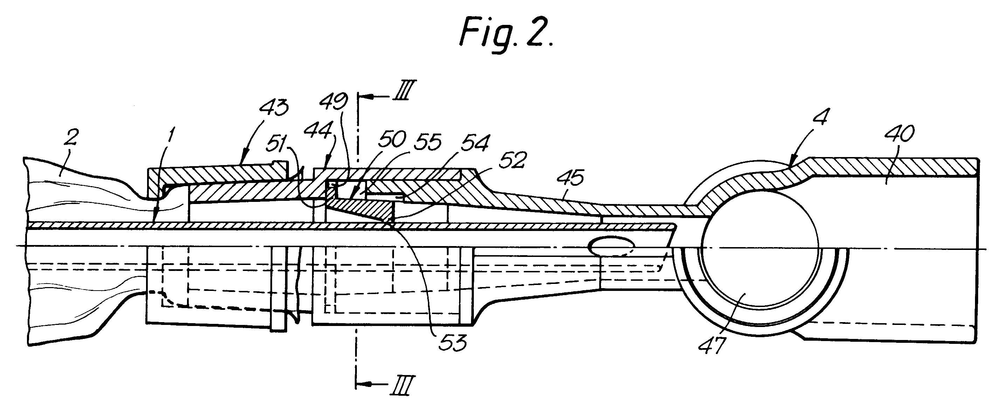

With reference first to Figures 1 to 3, the suction catheter assembly comprises an aspirating catheter 1 that extends within a flexible, protective sleeve 2 between a vacuum connecting member 3 and a patient connecting member 4.

The aspirating catheter 1 is of conventional construction having an outside diameter of about 4-5mm and a length of about 55cm. In the illustrated example, the catheter 1 has a single lumen 10 although catheters with multiple lumens for use in irrigation and/or oxygen supply could be used. At its machine or proximal end, the catheter 1 is secured to the vacuum connecting member 3.

The vacuum connecting member 3 is moulded from a rigid plastics material and has a bore (not shown) extending along it into one end of which the catheter 1 is bonded. The opposite end of the bore extends through a spigot 31, which, in use, is connected to tubing (not shown) extending to a vacuum or suction source 5. The vacuum connecting member 3 includes a conventional manually-operated valve 32, which normally prevents flow through the connecting member 3 and catheter 1 but which can be pressed down by the user to open the valve and connect the lumen 10 of the catheter to the suction source 5.

The proximal end of the sleeve 2 is secured to the vacuum connecting member 3 beneath a collar 33 secured to the distal end of the vacuum connecting member. The distal end of the sleeve 2 is similarly secured to the patient connecting member 4 by means of a collar 43 which is secured on a proximal extension piece 44 that is in turn joined to the main body 45 of the patient connecting member.

The patient connecting member 4 is of generally cruciform shape, having a female luer coupling 40 at its distal or patient end, which is aligned with the axis of the connecting member and with the proximal extension 44. The coupling 40 is adapted to be connected to a cooperating coupling (not shown) on the end of a tracheal tube. Two side ports 46 and 47 extend at right angles to the axis of the connecting member, directly opposite one another, about midway along the length of the connecting member. These two side ports 46 and 47 communicate directly with the interior of the coupling 40 and are used in the conventional manner to connect with ventilation apparatus. One port may be used for inhalation gas and the other port used for exhalation gas. Alternatively, one of the ports 47 may be closed by a cap 48 and inhalation and exhalation both be effected through the other port 46.

Within the patient connecting member 4 there is a novel sealing device and one-way valve 50. This takes the form of a resilient, generally tubular member having a flexible, external, annular flange 51 at its proximal end that is trapped between the main body 45 and a shoulder 49 in the proximal extension piece 44. The shoulder 49 faces distally of the assembly and is contacted by the proximal surface of the flange 51. The proximal end of the main body 45 has one or more cut-away portions 55 so that the flange 51 can be lifted away from the shoulder 49, into these portions, by elevated gas pressure on the proximal side of the flange. At its distal end, the valve 50 has an internally-extending wiper seal 52 defining a circular aperture 53 through which the catheter 1 extends and to which a sliding seal is formed. Externally, the valve 50 has a shallow taper of about 2-3°, so that it has a smaller diameter at its distal end. The distal surface of the flange 51 is exposed to gas pressure on the distal side of the valve by means of the cut-away portions 55 and passages 54 formed around the inside of the proximal end of the main body 45 of the patient connecting member. Internally, the valve 50 has a steeper taper of about 13-14° so that it has a smaller internal diameter and a greater wall thickness at its distal end. The interior of the valve 50 is exposed to gas pressure on the proximal side of the valve.

In operation, the coupling 40 of the connecting member 4 is secured to a coupling 6 on the end of a tracheal tube 7 and its side ports 46 and 47 are connected to a ventilator. The vacuum coupling member 3 is connected to the suction source 5 but, as long as the manual valve 32 remains unactuated, no suction is applied to the catheter 1. While mechanical ventilation takes place, there is raised, positive pressure within the patient connecting member 4 (on the distal side of the valve 50) which forces the distal side of the flange 51 against the shoulder 49, thereby improving the seal of the flange on the shoulder and blocking gas flow into the sleeve 2.

When aspiration of fluid from the trachea or bronchi is required, the user grips the catheter 1 through the sleeve 2 and pushes it forwardly so that the distal, patient end of the catheter is advanced through the connecting member 4 and into the tracheal tube 7. When the catheter 1 has been inserted to the desired depth, the user depresses the valve 32 so that the catheter is connected to the suction source 5 and fluid in the vicinity of the tip of the catheter is sucked into the catheter and removed. During aspiration, ventilation of the patient occurs normally. When aspiration is complete, the catheter 1 is pulled back into the sleeve 2, the assembly remaining attached to the tracheal tube connector 6 so that it can be reused when necessary.

Although the sliding seal of the valve 50 with the catheter 1 is effective to prevent any significant flow of gas into the sleeve 2, there may be some seepage of gas around the catheter, especially when the catheter is being manipulated during aspiration. The valve 50 is, however, effective to allow any gas trapped inside the sleeve 2 to escape when pressure on its proximal side exceeds that on the distal side. The action of gripping the sleeve 2 and advancing the catheter 1 has the effect of compressing any trapped gas in the sleeve, thereby increasing its pressure and allowing it to open the valve by forcing the flange 51 away from the shoulder 49 in the cut-away portions 55. The valve 50, therefore, acts as a one-way valve, allowing flow only distally of the valve. In this way, build-up of gas in the sleeve 2 is prevented since the sleeve 2 can vent at relatively low pressure. Because the venting occurs into the connecting member 4, the vented gas is carried to the ventilation system without any risk of cross contamination to the clinician.

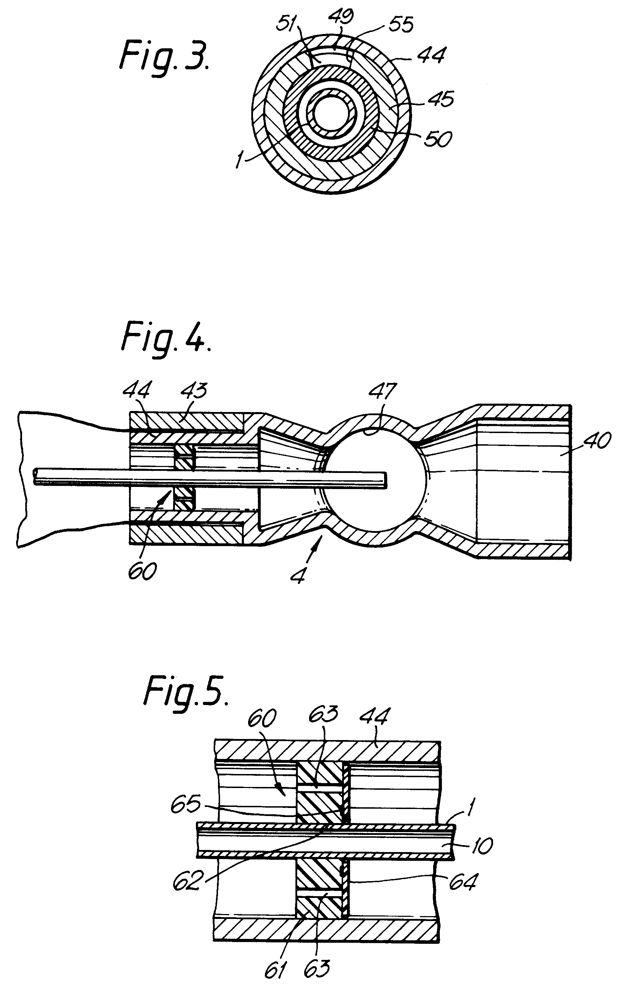

Various modifications are possible to the embodiment described. For example, the valve could be of the form shown in Figures 4 and 5. In this arrangement, the sealing device and one-way valve 60 takes the form of a disc 61 of a stiff but resilient material, such as a plastics, which is about 5mm thick and is bonded to the inside of the connecting member within the proximal extension 44 or at some other point proximally of the ventilation side ports 46 and 47. The disc 61 has a circular central aperture 62 which is a close sliding fit about the outside of the catheter 1 so as to form a sliding seal with its surface. The disc 61 is also provided with several smaller peripheral apertures 63 which extend through its thickness. The sealing device 60 is completed by a displaceable member in the form of a thin annular diaphragm 64 of a flexible gas-impervious plastics material. The diaphragm 64 is slightly smaller in external diameter than the disc 61 and has a central aperture that is slightly larger than the aperture 62 of the disc. The diaphragm 64 is bonded, such as by welding, solvent or adhesive, around its central aperture to the distal face of the disc 61 to form an annular bond 65. Outwardly of this bond 65, the diaphragm 64 overlies the peripheral apertures 63 and is unattached to the disc 61. The nature of the diaphragm 64 is such that it will remain flat against the surface of the disc for any orientation of the assembly but it can be deflected readily away from the disc by positive gas pressure through the apertures 63.

The one-way valve 50 or 60 could be provided in one part of a patient connecting member, with the side ports 46 and 47 and the female luer coupling 40 being provided in a separate part that is coupled together with the first part during use.

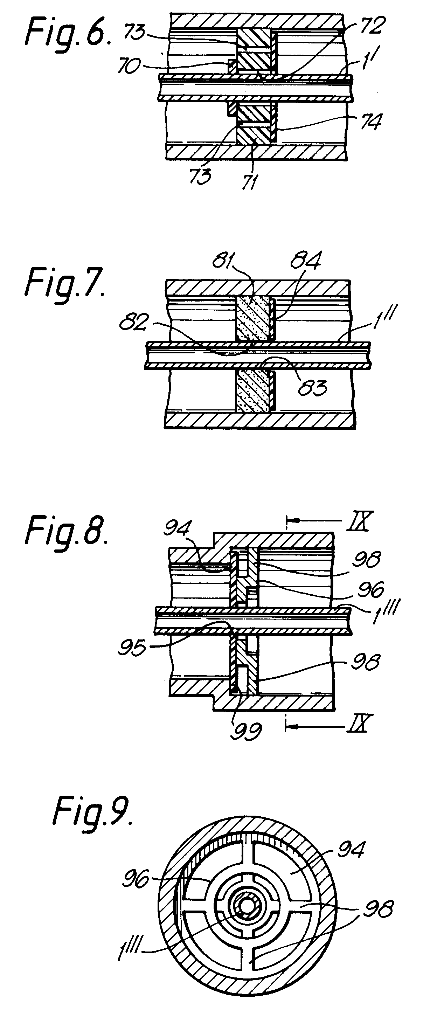

The one-way valve and the sliding seal with the catheter could be formed by separate components, as shown in Figure 6. In this arrangement, the sliding seal with the catheter 1' is provided by a gasket 70 secured to one side of a rigid valve disc 71. The valve disc 71 is similar to the disc 61 but has a larger central aperture 72 to allow freer movement of the catheter. A diaphragm 74 covers peripheral apertures 73 through the disc and seals them closed in the same way as described above.

Another modification is shown in Figure 7. In this, a disc 81 is made from a material that is porous through its thickness. A non-porous film 82 may be provided through a central aperture 83 to form an effective sliding seal with the catheter 1''. A flexible annular diaphragm 84 is attached only around its inner edge to the distal face of the disc 81 so that gas can flow through the disc in the distal direction but is impeded in a proximal direction.

In the arrangement shown in Figures 8 and 9, a diaphragm 94 acts as both a valve member and as a sliding seal with the catheter 1'''. The diaphragm 94 has a central aperture 95 that is a sliding seal with the outside of the catheter, the diaphragm being supported centrally by a skeletal support 96 that is secured to the housing of the patient connecting member 4 by means of radially extending arms 98. An internal annular step 99 is formed around the inside of the housing, the step facing distally of the assembly and the outer peripheral region of the diaphragm 94 being seated on this step. The edge of the diaphragm 94 is deformed away from the step 99 by pressure from within the sleeve but, in normal use, the pressure from the respiration system forces the edge of the diaphragm in a proximal direction, more firmly against the step so that air can only flow in one direction.

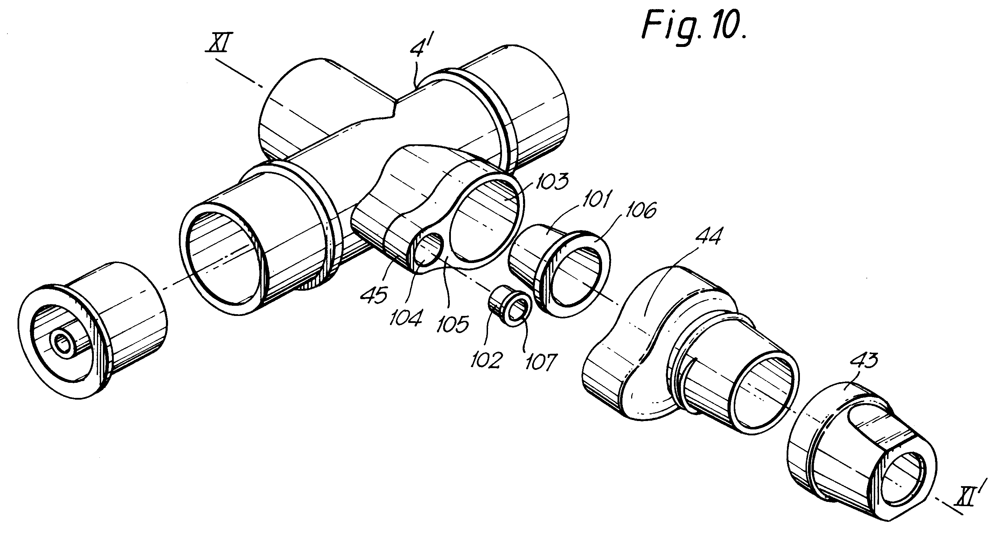

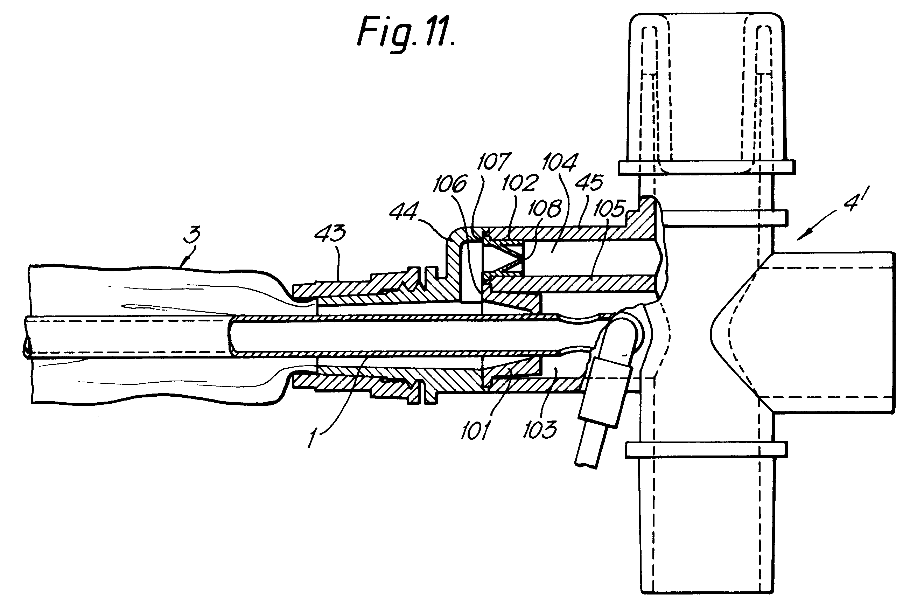

Figures 10 and 11 show another modification of the patient connecting member 4'. The housing of the patient connecting member 4' includes a small by-pass channel 104 in addition to a main passage 103 through which the aspiration catheter is advanced. A stationary sliding seal or wiper/seal member 101 is provided in the main passage 103 sealingly engaging the outer surface of the aspirating catheter 1. A one-way, V-shape check valve, such as a duck-bill valve 102 is mounted in the patient connecting member 4' to allow passage of entrapped air from the protective sleeve 3 into the patient connecting member 4'. The one-way valve 102 is located in the by-pass channel 104 so that air from the sleeve flows along a path defined by the by-pass channel 104 and into the patient connecting member 4'. The by-pass channel 104 is formed between the outer wall of the housing (main body 45 and extension 44) and an inner wall 105 substantially surrounding the seal 101 and coaxial with the outer wall of the housing. As shown in Figure 11, the one-way valve 102 positioned in the by-pass channel 104 is substantially parallel with the wiper/seal 101. The rear ends 106 and 107 of the wiper seal 101 and the one-way valve 102 respectively are substantially aligned with each other. The by-pass channel 104 communicates with the main passage 103 at one end at the location facing the rear end 106 of the wiper/seal member 101 and at the other end at the location facing the front end 108 of the wiper/seal.

In operation, advancing the catheter 1 into the patient compresses the protective sleeve 3. This compression pressurizes the air inside the sleeve causing the one-way valve 102 to open and allow air to flow out of the sleeve into the T-piece and ventilation circuit. A certain amount of residual air will remain in the sleeve at atmospheric pressure so that the sleeve does not cling to the catheter when it is withdrawn from the patient. This is an important advantage over prior valves and facilitates manoeuvring of the catheter. The one-way valve prevents air from the ventilator entering the sleeve, thereby reducing the risk that the sleeve will be inflated.

## Claims

1. A suction catheter assembly for use in removing undesirable fluid from a patient, the catheter assembly including an aspirating catheter (1) having a distal end to be inserted in a patient, a vacuum connecting member (3) located in the vicinity of the proximal end of the aspirating catheter, a patient connecting member (4, 4') surrounding the aspirating catheter in the vicinity of the distal end of the aspirating catheter and having a sliding seal (52, 62) with the outside of the aspirating catheter, a protective sleeve (2) surrounding at least the majority of the length of the catheter (1) and extending between the vacuum connecting member (3) and the patient connecting member (4, 4'), the protective sleeve (2) permitting the distal end of the catheter to be extended from the protective sleeve into the patient and to be withdrawn from the patient, the patient connecting member (4, 4') including a one-way valve (50, 60, 102) permitting gas flow through it from inside the sleeve (2) and into the patient connecting member but preventing any substantial gas flow through the one-way valve into the sleeve such that any gas trapped in the sleeve can escape into the patient connecting member through the one-way valve, characterised in that the one-way valve (50, 60, 102) has a one-way gas passage from inside the sleeve other than via the sliding seal (52, 62, 72, 82) and the outside of the catheter (1).

2. A suction catheter assembly according to Claim 1, characterised in that the one-way valve is a duck-bill valve (102).

3. A suction catheter assembly according to Claim 1 or 2, characterised in that the patient connecting member (4') includes a main passage (103) through which the aspirating catheter (1) is advanced and a by-pass channel (104), and that the one-way valve (102) is located in the by-pass channel (104).

4. A suction catheter assembly according to Claim 3, characterised in that the by-pass channel (104) is substantially parallel with the main passage (103).

5. A suction catheter assembly according to Claim 3 or 4, characterised in that the by-pass channel (104) communicates with the main passage (103) at one end at a location facing the sleeve (2) and the rear of the sliding seal (101) and at the other end at a location facing the front of the sliding seal (101).

6. A suction catheter assembly according to Claim 1, characterised in that the sliding seal (52) and one-way valve are provided by a resilient tubular member (50) having a flange (51) at its proximal end and an internal wiper seal (52) with the catheter (1) at its distal end, that the assembly includes a shoulder (49) that faces distally of the assembly, and that the proximal surface of the flange (51) lies against the shoulder (49) and the distal surface of the flange is exposed to gas pressure in the patient connecting member (4) distally of the tubular member (50) such that the flange (51) is forced into closer contact with the shoulder (49) by gas pressure on the distal side of the tubular member and is lifted away from the shoulder by gas pressure on the proximal side of the tubular member.

7. A suction catheter assembly according to Claim 1, characterised in that the one-way valve includes a disc (61, 71) with a central aperture (62, 72) through which the catheter (1) extends, the disc having at least one peripheral aperture (63, 73) therethrough providing the one-way gas passage, and a displaceable member (64, 74) overlying a distal face of the disc (61, 71) in the region of the peripheral aperture (63, 73) such that gas pressure on the proximal side of the disc is effective to displace the displaceable member (64, 74) away from the peripheral aperture so as to allow gas flow through the peripheral aperture.

8. A suction catheter assembly according to Claim 1, characterised in that the one-way valve includes a porous disc (81) providing the one-way gas passage, that the disc (81) has an aperture (83) through which the catheter (1) extends, and a flexible diaphragm (84) overlying a distal face of the disc (81) such that gas pressure on the proximal side of the disc is effective to displace a part of the diaphragm (84) away from the disc so as to allow gas flow through the disc, and such that gas pressure on the distal side of the disc (81) is effective to urge the diaphragm against the disc and prevent gas flow therethrough.

9. A suction catheter assembly according to any one of the preceding claims, characterised in that the patient connecting member includes a patient end coupling (4, 4') aligned with the aspirating catheter (1), that the patient end coupling (4, 4') is adapted to be coupled with a tracheal tube (7), that the patient end coupling has two side ports (46, 47) communicating with the interior of the patient end coupling on the distal side of the one-way valve (50, 60, 102), and that the two side ports are adapted for connection to ventilation apparatus such that the patient can be aspirated without interruption of ventilation.

10. A suction catheter assembly according to any one of the preceding claims, characterised in that the vacuum connecting member (3) includes a manually-operable valve (32), and that the manually-operable valve normally blocks fluid flow along the aspirating catheter (1) but can be manually opened to permit fluid flow along the catheter.

## Patentansprüche

1. Absaugkatheteraufbau zum Entfernen unerwünschter Fluide aus einem Patienten, wobei der Katheteraufbau einen Absaugkatheter (1) aufweist, der ein fernes Ende zum Einführen in einen Patienten besitzt, ein Vakuumanschlußstück (3), welches sich in der Nähe des nahen Endes des Absaugkatheters befindet, ein Patientenanschlußstück (4, 4'), welches den Absaugkatheter in der Nähe des fernen Endes des Absaugkatheters umschließt und eine Gleitdichtung (52, 62) mit der Außenseite des Absaugkatheters besitzt, einen Schutzhüllschlauch (2), welcher zumindest den größten Teil der Länge des Katheters (1) umgibt und zwischen dem Vakuumanschlußstück (3) und dem Patientenanschlußstück (4, 4') verläuft, wobei der Schutzhüllschlauch (2) es gestattet, daß das ferne Ende des Katheters aus dem Schutzhüllschlauch in den Patienten hinein verlängert und aus dem Patienten zurückgezogen wird, das Patientenanschlußstück (4, 4') ein Ein-Weg-Ventil (50, 60, 102) besitzt, welches einen Gasfluß durch sich von der Innenseite des Hüllschlauches (2) in das Patientenanschlußstück gestattet, aber jeden wesentlichen Gasfluß durch das Ein-Weg-Ventil in den Hüllschlauch verhindert, so daß jegliches im Hüllschlauch eingeschlossene Gas in das Patientenanschlußstück durch das Ein-Weg-Ventil entweichen kann, **dadurch gekennzeichnet**, daß das Ein-Weg-Ventil (50, 60, 102) eine Einweg-Gaspassage von der Innenseite des Hüllschlauches besitzt, die anders als über die Gleitdichtung (52, 62, 72, 82) und die Außenseite des Katheters (1) verläuft.

2. Saugkatheteraufbau nach Anspruch 1, **dadurch gekennzeichnet**, daß das Ein-Weg-Ventil ein Entenschnabelventil (102) ist.

3. Saugkatheteraufbau nach Anspruch 1 oder 2, **dadurch gekennzeichnet**, daß das Patientenanschlußstück (4') eine Hauptpassage (103) besitzt, durch die der Saugkatheter (1) vorgeschoben wird, und einen By-Pass-Kanal (104), und daß das Ein-Weg-Ventil (102) sich im By-Pass-Kanal (104) befindet.

4. Saugkatheteraufbau nach Anspruch 3, **dadurch gekennzeichnet**, daß der By-Pass-Kanal (104) im wesentlichen parallel mit der Hauptpassage (103) ist.

5. Saugkatheteraufbau nach Anspruch 3 oder 4, **dadurch gekennzeichnet**, daß der By-Pass-Kanal (104) mit der Hauptpassage (103) an einem Ende in Verbindung steht an einer Stelle, die zum Hüllschlauch (2) und zur Rückseite der Gleitdichtung (101) weist und am anderen Ende an einer Stelle, die zur Vorderseite der Gleitdichtung (101) weist.

6. Saugkatheteraufbau nach Anspruch 1, **dadurch gekennzeichnet**, daß die Gleitdichtung (52) und das Ein-Weg-Ventil durch ein federndes, röhrenförmiges Element (50) vorgesehen werden, welches einen Flansch (51) an seinem nahen Ende und eine innenliegende Wischdichtung (52) mit dem Katheter (1) an seinem fernen Ende besitzt, daß die Vorrichtung eine Schulter (49) besitzt, welche von der Vorrichtung weg weist, und daß die nahe Oberfläche des Flansches (51) gegen die Schulter (49) anliegt und die ferne Oberfläche des Flansches Gasdruck im Patientenanschlußstück (4) auf der fernen Seite des röhrenförmigen Elementes (50) ausgesetzt ist, so daß der Flansch (51) durch Gasdruck auf der fernen Seite des röhrenförmigen Elementes in engeren Kontakt mit der Schulter (49) gepreßt wird und von der Schulter durch Gasdruck auf der nahen Seite des röhrenförmigen Elementes abgehoben wird.

7. Saugkatheteraufbau nach Anspruch 1, **dadurch gekennzeichnet**, daß das Ein-Weg-Ventil eine Scheibe (61, 71) mit einer zentralen Öffnung (62, 72) besitzt, durch welche der Katheter (1) verläuft, wobei die Scheibe zumindest eine periphere Öffnung (63, 73) besitzt, durch welche die Einweggaspassage gestattet wird, und ein entfernbares Element (64, 74), welches eine ferne Oberfläche der Scheibe (61, 71) im Bereich der peripheren Öffnung (63, 73) überlagert, so daß Gasdruck auf der nahen Seite der Scheibe zur Entfernung des entfernbaren Elementes (64, 74) von der peripheren Öffnung weg wirksam ist, um Gasfluß durch die periphere Öffnung zu gestatten.

8. Saugkatheterautbau nach Anspruch 1, **dadurch gekennzeichnet**, daß das Ein-Weg-Ventil eine poröse Scheibe (81) besitzt, welche die Einweggaspassage bietet, daß die Scheibe (81) eine Öffnung (83) besitzt, durch welche der Katheter (1) verläuft, und ein flexibles Diaphragma (84), welches eine ferne Fläche der Scheibe (81) überlagert, so daß Gasdruck auf die nahe Seite der Scheibe wirksam ist, um einen Teil des Diaphragmas (84) von der Scheibe weg zu entfernen, um einen Gasfluß durch die Scheibe zu gestatten, und daß Gasdruck auf der fernen Seite der Scheibe (81) wirksam ist, um das Diaphragma gegen die Scheibe zu drücken und einen Gasfluß hierdurch zu verhindern.

9. Absaugkatheteraufbau nach einem der vorangehenden Ansprüche, **dadurch gekennzeichnet**, daß das Patientenanschlußstück eine Patientenendkopplung (4, 4') besitzt, welche mit dem Absaugkatheter (1) fluchtet, daß die Patientenendkopplung (4, 4') angeschlossen ist, um mit einem Trachealtubus (7) gekoppelt zu werden, daß die Patientenendkopplung zwei Seitenanschlüsse (46, 47) besitzt, welche mit dem Inneren der Patientenendkopplung auf der fernen Seite des Ein-Weg-Ventils (50, 60, 102) in Verbindung stehen, und daß die beiden Seitenanschlüsse zur Verbindung an den Beatmungsapparat angepaßt sind, so daß der Patient ohne Unterbrechnung der Beatmung abgesaugt werden kann.

10. Saugkatheteraufbau nach einem der vorangehenden Ansprüche, **dadurch gekennzeichnet**, daß das Vakuumanschlußstück (3) ein handbedienbares Ventil (32) besitzt und daß das handbedienbare Ventil normalerweise einen Fluidfluß entlang des Absaugkatheters (1) unterbindet, aber manuell geöffnet werden kann, um einen Fluidfluß entlang des Katheters zu gestatten.

## Revendications

1. Ensemble formant un cathéter de succion à utiliser pour éliminer des fluides indésirables d'un patient, l'ensemble cathéter comprenant un cathéter de succion (1) ayant une extrémité distale à insérer dans un patient, un élément de connexion au vide (3) disposé à proximité de l'extrémité proximale du cathéter de succion, un élément de connexion au patient (4, 4') entourant le cathéter de succion à proximité de l'extrémité distale du cathéter de succion et comprenant un joint d'étanchéité glissant (52, 62) sur l'extérieur du cathéter de succion, un manchon protecteur (2) entourant au moins la plus grande partie de la longueur du cathéter (1) et s'étendant entre l'élément de connexion à un appareil à vide (3) et l'élément de connexion au patient (4, 4'), le manchon protecteur (2) permettant à l'extrémité distale du cathéter d'être amenée du manchon protecteur dans le patient et d'être retirée du patient, l'élément de connexion au patient (4, 4') comprenant une valve unidirectionnelle (50, 60, 102) permettant une circulation de gaz à travers celle-ci depuis l'intérieur du manchon protecteur (2) vers l'intérieur de l'élément de connexion au patient mais empêchant toute circulation substantielle de gaz à travers la valve unidirectionnelle vers l'intérieur du manchon protecteur, de sorte que tout le gaz retenu dans le manchon peut s'échapper dans l'élément de connexion au patient à travers la valve unidirectionnelle, caractérisé en ce que la valve unidirectionnelle (50, 60, 102) comporte un passage de gaz unidirectionnel depuis l'intérieur du manchon protecteur autre que par le joint d'étanchéité glissant (52, 62, 72, 82) et l'extérieur du cathéter (1).

2. Ensemble formant un cathéter de succion selon la revendication 1, caractérisé en ce que la valve unidirectionnelle est une valve à bec de canard (102).

3. Ensemble formant un cathéter de succion selon la revendication 1 ou 2, caractérisé en ce que l'élément de connexion au patient (4') comporte un passage principal (103) à travers lequel le cathéter de succion (1) débouche et un canal de dérivation (104), et en ce que la valve unidirectionnelle (102) est disposée dans le canal de dérivation (104).

4. Ensemble formant un cathéter de succion selon la revendication 3, caractérisé en ce que le canal de dérivation (104) est sensiblement parallèle au passage principal (103).

5. Ensemble formant un cathéter de succion selon la revendication 3 ou 4, caractérisé en ce que le canal de dérivation (104) communique avec le passage principal (103) à une extrémité à un endroit en regard du manchon protecteur (2) et de l'arrière du joint d'étanchéité glissant (101) et à l'autre extrémité à un endroit en regard de l'avant du joint d'étanchéité glissant (101).

6. Ensemble formant un cathéter de succion selon la revendication 1, caractérisé en ce que le joint d'étanchéité glissant (52) et la valve unidirectionnelle sont formés par un élément tubulaire élastique (50) comportant une collerette (51) à son extrémité proximale et un joint d'étanchéité frotteur interne (52) avec le cathéter (1) à son extrémité distale, en ce que l'ensemble comprend un épaulement (49) qui fait face de façon distale à l'ensemble, et en ce que la surface proximale de la collerette (51) repose contre l'épaulement (49) et la surface distale de la collerette est exposée à la pression du gaz dans l'élément de connexion au patient (4) éloignée de l'élément tubulaire (50) de sorte que la collerette (51) est forcée en contact étroit avec l'épaulement (49) par la pression du gaz sur la face distale de l'élément tubulaire et est décollée de l'épaulement par la pression du gaz sur la face proximale de l'élément tubulaire.

7. Ensemble formant un cathéter de succion selon la revendication 1, caractérisé en ce que la valve unidirectionnelle comprend un disque (61, 71) comportant une ouverture centrale (62, 72) à travers lequel s'étend le cathéter (1), le disque comportant au moins un orifice périphérique (63, 73) fournissant le passage unidirectionnel du gaz, et un élément mobile (64, 74) recouvrant une face distale du disque (61, 71) dans la région de l'ouverture périphérique (63, 73) de sorte que la pression du gaz sur la face proximale du disque est efficace pour écarter l'élément mobile (64, 74) de l'ouverture périphérique afin de permettre la circulation du gaz à travers l'ouverture périphérique.

8. Ensemble formant un cathéter de succion selon la revendication 1, caractérisé en ce que la valve unidirectionnelle comprend un disque poreux (81) fournissant le passage unidirectionnel du gaz, en ce que le disque (81) comporte un ouverture (83) à travers laquelle s'étend le cathéter (1), et un diaphragme flexible (84) recouvrant une face distale du disque (81) de sorte que la pression du gaz sur la face proximale du disque est efficace pour écarter une partie du diaphragme (84) du disque afin de permettre la circulation du gaz à travers le disque, et de sorte que la pression du gaz sur la face distale du disque (81) est efficace pour pousser le diaphragme contre le disque et empêcher la circulation du gaz à travers celui-ci.

9. Ensemble formant un cathéter de succion selon l'une quelconque des revendications précédentes, caractérisé en ce que l'élément de connexion au patient comprend un couplage à l'extrémité côté patient (4, 4') alignée avec le cathéter de succion (1), en ce que le couplage à l'extrémité patient (4, 4') est adapté pour être coupé à un tube trachéal (7), en ce que le couplage à l'extrémité patient comporte deux orifices latéraux (46, 47) communiquant avec l'intérieur du couplage à l'extrémité patient sur la face distale de la valve unidirectionnelle (50, 60, 102), et en ce que les deux orifices latéraux sont adaptés pour être connectés à un appareil de ventilation afin que le patient puisse être aspiré sans interruption de la ventilation.

10. Ensemble formant un cathéter de succion selon l'une quelconque des revendications précédentes, caractérisé en ce que l'élément de connexion à un appareil à vide (3) comprend une vanne à commande manuelle (32), et en ce que la vanne à commande manuelle normalement bloque la circulation du fluide le long du cathéter de succion (1) mais peut être ouverte manuellement pour permettre la circulation du fluide le long du cathéter.
